# EUROPEAN PATENT SPECIFICATION

(11) **EP 4 501 208 B1**
(45) Date of publication and mention of the grant of the patent: **26.08.2026**
(21) Application number: 24202782.9
(22) Date of filing: 31.01.2023
(51) Int. Cl.: A61B 1/00, A61B 1/12, A61B 90/70

(54) **A SYSTEM FOR SUPPLYING A PROCESS MEDIUM TO MEDICAL ARTICLES HAVING MULTIPLE CHANNELS**
SYSTEM ZUR ZUFUHR EINES PROZESSMEDIUMS ZU MEDIZINISCHEN ARTIKELN MIT MEHREREN KANÄLEN
SYSTÈME POUR FOURNIR UN MILIEU DE TRAITEMENT À DES ARTICLES MÉDICAUX AYANT DE MULTIPLES CANAUX

(43) Date of publication of application: 05.02.2025
(62) Document number(s) of the earlier application(s) in accordance with Art. 76 EPC: 23154119.4 / 4 410 177
(73) Proprietor: Getinge Disinfection AB, 351 15 Växjö (SE)
(72) Inventor: ENGDAHL, Erik, 342 64 Växjö (SE); ENG STENSSON, Alexander, 352 64 Växjö (SE)
(74) Representative: Zacco Sweden AB

(56) References cited:
- EP-A1- 3 586 788
- EP-A2- 1 946 692
- EP-B1- 0 483 059

## Description

### TECHNICAL FIELD

The present disclosure relates to a system for supplying a process medium to medical articles having multiple channels for washing, disinfection, and drying of the channels.

### BACKGROUND ART

Medical articles that have multiple channels are frequently used in hospitals, such as in surgical departments and similar facilities. The channels may be used for providing access to body organs without any, or with only limited, surgical intervention. For instance, different types of endoscopes are examples of such medical articles.

Such a medical article should be washed/disinfected after it has been used, so that it can be re-used at another occasion. Washing and/or disinfecting may for example be performed in combined medical washer-disinfectors that are typically provided at surgical departments, central sterile supply departments, and similar facilities. The medical washer-disinfectors are configured to allow the medical articles to be washed on the outside as well internally, i.e. inside the channels. In order to wash and/or disinfect the channels each individual channel is manually connected to a respective connector in the medical washer-disinfector. Water, disinfection chemicals, and/or detergent can then be supplied through the connectors to the respective channels. After the treatment of the medical article has been completed, the channels are manually disconnected. Normally, the medical articles are then transferred to a drying cabinet in order to dry the medical articles, externally as well as internally. Again, each channel is manually connected to a connector of the drying cabinet, this time to allow air to be circulated in the channels for drying the channels. Eventually, the channels are manually disconnected from the connectors of the drying cabinet. The repeated manual connection and disconnection is time-consuming. EP 0 483 059 B1 discloses a washing machine which carries our both cleaning and testing of devices to be cleaned, in particular endoscopes. The washing machine has means for helping to wash through the channels by means of pneumatic impulsions. The device to be cleaned is connected automatically to testing and cleaning conduits.

### SUMMARY OF THE DISCLOSURE

An object of the present invention is to mitigate the above mentioned drawbacks of conventional washers-disinfectors. This and other objects, which will become apparent in the following, are accomplished by the invention as defined in claim 1. Some non-limiting exemplary embodiments are presented in the dependent claims.

The applicants have determined that channels of a medical article should be connected to a removable coupling device, which may function as an interface between the channels and a medical processing device (such as a washer-disinfector or a drying cabinet). The applicants have also determined that the actual act of connecting the channels to the removable coupling device can be performed outside of the medical processing device and then, when the removable coupling device (with the channels now being connected) has been inserted into the medical processing device, nozzles of the medical processing device may be moved into the removable coupling device in order to quickly form a tight seal, and allow a process medium to be passed from the nozzles via the intermediate removable coupling device and into the channels. Advantageously, when the medical article is to be moved from one medical processing device to another, such as from a washer-disinfector to a drying cabinet, there is no need to disconnect the channels. Instead, the removable coupling device can simply be moved, with the channels still connected, from one medical processing device to another. By providing nozzles which are movable so that they can engage with the removable coupling device and then be moved back in and out of the way when not in use, a smooth loading and unloading of medical articles may be achieved, without risk of damaging the nozzles, catching objects on the nozzles, or operator injury on a protruding nozzle.

Thus, according to the present invention, there is provided a system for supplying a process medium to medical articles having multiple channels according to claim 1, the system comprising:
- a medical processing device configured to receive medical articles to be subjected to a process medium, the medical processing device comprising a plurality of nozzles, each nozzle being movable between a retracted position and an advanced position,
- a removable coupling device comprising a plurality of fluid passages, each fluid passage being configured to be connectable to a respective channel of a medical article having multiple channels before the removable coupling device is inserted into the medical processing device, wherein the removable coupling device is configured to be positioned at a docking location inside the medical processing device so that movement of the nozzles from said retracted position to said advanced position causes at least some of said nozzles, in particular all of said nozzles, to become engaged with respective fluid passages of the removable coupling device, thereby enabling the medical processing device to supply the process medium from the nozzles, via the engaged fluid passages of the removable coupling device, into any channel of the medical article connected to the removable coupling device.

The medical processing device may be used for washing (cleaning) and/or disinfecting the medical articles. It should be understood that in this disclosure, the terms "washing" and "cleaning" are considered interchangeable and that the system is for both.

By providing a removable coupling device and configuring the nozzles of a medical processing device to be advanceable into engagement with the removable coupling device, a time-saving and robust system is achieved. As indicated previously, by allowing the nozzles to be moved to the advanced position for processing of medical articles and then be returned to a retracted position (preferably retracted within a wall or side surface of the device chamber), an efficient docking of the removable coupling device is achieved without compromising the integrity of the nozzles. By having movable nozzles, it is possible to store them (in the retracted position) in such manner that they are protected from any inadvertent collision with other components, such as during loading and unloading of medical articles in the medical processing device. In particular, the plurality of nozzles may be advanceable separately from the structures and surfaces surrounding and in between the plurality of nozzles.

In some preferred embodiments of the retracted position, the plurality of nozzles are withdrawn so that they are behind a plane of the chamber surfaces immediately surrounding the plurality of nozzles and/or they are fully withdrawn from the inside of the device chamber.

As indicated above, the removable coupling device may suitably be usable in connection with various different types of medical processing devices. For example, in this disclosure, a medical processing device may be a washer, a disinfector, a combined washer-disinfector, a dedicated endoscope reprocessor or endoscope washer-disinfector, a drying cabinet, a combined drying and storage cabinet, etc. Furthermore, although the system may be used for supplying process medium to any suitably medical article having multiple channels, it is envisaged that the system may in particular be used for supplying a process medium to endoscopes having multiple channels. It should nevertheless be understood that although the beneficial time-saving discussed in the present disclosure is particularly relevant for medical articles having a plurality of channels, the system of the present disclosure could also be used for supplying process medium to a medical article having a single internal channel. Thus, it should be understood that any fluid passage of the removable coupling device not connected to a respective internal channel of a medical article may for example be used for flushing process medium externally of the medical article and/or may be partly restricted so as to reduce the flow through that fluid passage without causing unwanted pressure effects in the system (assuming all nozzles are engaged with a respective fluid passage and all nozzles supply process medium).

Although in some examples the movements of the nozzles may be individually controllable and/or the supply of process medium from the nozzles may be individually controllable, in other examples the nozzles may suitably be configured to be moved collectively as that may simplify the overall controlling of the nozzle movement. Furthermore, the all nozzles may suitably be collectively provided with process medium from a common process medium source. For example, all of the nozzles (and the corresponding connected lumens) might be simultaneously provided first with one or more liquids for washing (cleaning) and/or disinfection, and then simultaneously provided with a gas (such as air) for drying the lumens. There might optionally by an alcohol step between the first liquid step and the gas step. In such embodiments, there is no need for individually controllable valves provided between the process medium source and the nozzles.

Depending on the medical processing device, different types of process medium may be supplied. A process medium may be any suitable type of fluid. Examples of such fluids are liquid and gas. A liquid may, for instance, be water (with or without a detergent or a disinfectant), alcohol, or any other suitable liquid. A gas may, for instance, be air. Thus, in the case of the medical processing device being in the form of a washer-disinfector, a liquid may suitably be used as process medium, while in the case of a drying cabinet, the process medium may suitably be air.

The internal channels of the medical article may sometimes be referred to as lumens. Without being bound to a specific terminology for the present inventive concept, it should be understood that channels/lumens may both refer to passages inside a medical article, such as inside an endoscope.

According to at least some exemplary embodiments, the medical processing device comprises a docking unit, the docking unit comprising a plurality of compartments, one for each nozzle, wherein
- in said retracted position, each nozzle is located at least partially inside its respective compartment, in particular fully inside its respective compartment, and
- in said advanced position, each nozzle projects out from its respective compartment.

By providing each nozzle movable into and out from a respective compartment separately from the docking unit, the nozzles are well protected and are not obtrusive when not in use. Furthermore, the provision of individual compartments facilitates any sealing around each nozzle to prevent process medium from reaching any sensitive components of the medical processing device. Examples of sealing means will be discussed later in this disclosure.

According to at least one exemplary embodiment, the nozzles each advance along a different respective central axis out and away from a side wall or docking unit of the medical processing device when moving into the advanced position. For instance, the nozzles may each advance out and away from the docking unit mentioned above.

According to at least one exemplary embodiment, in the retracted position the nozzles are each at least partially in a respective cavity of the medical processing device, and in the advanced position they each enter into a respective cavity of the removable coupling device. In the case of a docking unit being included in the medical processing device, said cavities of the medical processing device may correspond to the above-mentioned compartments of the docking unit, wherein each nozzle may in the retracted position be at least partially in a respective compartment of the docking unit. Said respective cavities of the removable coupling device may suitably form part of a respective one of said fluid passages of the removable coupling device.

According to at least some exemplary embodiments, said docking location for the removable coupling device is aligned with the docking unit such that when the removable coupling device is positioned at the docking location, each fluid passage is co-axially aligned with a respective nozzle. The docking location may suitably be defined by a holder, a receiving element, a bracket, an end stop, or any other suitable means which makes the docking location easily locatable when inserting the removable coupling device into the medical processing device. However, it should be understood that positioning of the removable coupling device in said docking location does not necessarily need to be facilitated by elements or features within the medical processing device, but may in some examples be facilitated by other elements, even external elements. For instance, rather than putting medical articles directly onto the floor of the internal chamber of the medical processing device, it is common practice to place medical articles in a load carrier, such as a basket or a tray, which is then inserted into the chamber of the medical processing device. Such a load carrier may, for example, be introduce into the chamber of the medical processing device by sliding it along rails in the chamber. Therefore, in at least some exemplary embodiments, the system may comprise a load carrier which has a predefined receiving portion to which the removable coupling device should be mounted. When the load carrier is subsequently fully introduced into the chamber of the medical processing device (with the removable coupling device mounted to the predefined receiving portion), the removable coupling device may thereby automatically arrive at the docking location within the medical processing device.

According to at least some exemplary embodiments, in the advanced position of the nozzles, the nozzles are configured to project into the fluid passages of the removable coupling device. A technical benefit may include that the design of the removable coupling device can be relative simple, including a plurality of through holes forming said fluid passages, and in some embodiments having no actively moving parts, or at least no parts which are required to move during the docking process. The nozzle-receiving side of the removable coupling device may therefore be substantially flat, with the fluid passages extending from the flat side through the removable coupling device to the opposite side. The nozzles may in this case be regarded as male connectors and the removable coupling device with its fluid passages may be regarded as forming female connectors. Although the above-mentioned configuration is advantageous, it should be understood that the general inventive concept may also be implemented by configuring the nozzles as female connectors which, when they are advanced, engage and surround respective male connectors that form the fluid passages of the removable coupling device.

According to at least one exemplary embodiment, each nozzle is provided with at least one seal configured to follow the movement of the nozzle. By having a seal around each nozzle the risk of fluid leaking back, past and around the nozzle, is reduced. Furthermore, control of the fluid flow rate and fluid pressure can be more accurate. In the case of the nozzle being in the form of a male connector, the seal is advantageously provided annularly around the enveloping surface of the nozzle. In the case of the nozzle being in the form of a female connector the seal could instead be provided along the inner perimeter of the nozzle.

According to at least one exemplary embodiment, each nozzle comprises a front seal and a rear seal, each having an inner periphery that is in encircling contact with the nozzle, wherein when the nozzle is in the advanced position and is engaged with a fluid passage, an outer periphery of the front seal seals against a circumferential wall portion of the engaged fluid passage, and an outer periphery of the rear seal seals against a circumferential wall portion of the compartment from which the nozzle projects. In this way, fluid flow rate and pressure control through via the fluid passages will become accurate due to the front seal, and the rear seal reduces the risk of fluid leaking into any sensitive areas of the medical processing device.

From an efficiency perspective it may be an advantage for a central sterile supply department or similar facility to have a plurality of removable coupling devices. For instance, when the staff is preparing a first medical article by connecting it to a first removable coupling device, there may already be a second medical article connected to a second removable coupling device under treatment inside a medical processing device (e.g. a washer-disinfector). At the same time a third removable coupling device with a connected third medical article may be simultaneously transported from that medical processing device to a another one (e.g. from a washer-disinfector to a drying cabinet). A fourth removable coupling device with a connected fourth medical article may at the same time be inside the other medical processing device, etc. Thus, it should be understood that, according to at least some exemplary embodiments, the system may comprise a plurality of removable coupling devices. It is conceivable to provide the one or more removable coupling devices of the system with sealing elements. However, considering the above examples in which the system may have be many more removable coupling devices than medical processing devices, it may be convenient and more cost-efficient to provide the nozzles of the medical processing devices with seals rather than equipping each one of the plurality of removable coupling devices with sealing elements. According to at least one exemplary embodiment, in the advanced position of the nozzles, when they have engaged the fluid passages, the nozzles prevent the removable coupling device from moving in a radial direction relative to the axial extension of any one of the nozzles. This is advantageous as a separate locking step or mechanism is not required against movement in said dimensions. As regards preventing movement of the removable coupling device in the axial direction, i.e. along the axial extension of any one of the nozzles, there may be various ways to achieve this. For instance, the removable coupling device may be firmly mountable to a load carrier such as a tray or a basket which is itself prevented from moving in that axial dimension by a wall of the chamber, or otherwise. Such firm mounting may also counter-act movement in the radial direction. Another locking against axial and/or rotational movement may be achieved by appropriate designing of the previously mentioned docking unit. The docking unit may have locking features that engage the removable coupling device when the removable coupling device arrives at its docking location.

Similarly to the above example, according to at least one exemplary embodiment, the removable coupling device comprises or is part of a structure for holding the medical article when the medical article is connected to the removable coupling device, in particular wherein the structure for holding the medical article comprises a basket, bin, box, tray, or platform. Thus, it should be understood that the present disclosure encompasses structures to which a removable coupling device may be mounted before treatment of a medical article and dismounted afterwards, and the present disclosure also encompasses integrated structures for holding the medical article.

According to the present invention, the medical processing device comprises an actuator, wherein the actuator is configured to move the nozzles between said retracted position and said advanced position. This is advantageous as the movement of the nozzles may suitably be motorized and/or automated.

According to the present invention, the actuator comprises a motor and a movable actuator part, wherein the motor is configured to drive the movable actuator part alternatingly in a forward and reverse motion along a first geometrical axis,
wherein one of said forward and reverse motions of the movable actuator part causes the nozzles to move transversely to said first geometrical axis from the retracted position to the advanced position, and
wherein the other one of said forward and reverse motions of the movable actuator part causes the nozzles to return from the advanced position to the retracted position. By configuring the movable actuator part to travel in one direction, and the nozzles to travel in another direction, a space-saving arrangement is achievable. For instance, the movable actuator part may travel vertically upwards and downwards along a vertical wall of the medical processing device, while the nozzles may be advanced and retracted in a horizontal direction to engage/disengage the fluid passages of the removable coupling device located in the chamber of the medical processing device. Thus, in a general sense, the motor may drive the movable actuator in one direction, which may be transferred to a perpendicular movement of the nozzles.

According to at least one exemplary embodiment, the actuator part comprises an inclined plane configured to interact with a nozzle-holding part, wherein movement of the actuator part in one of said forward and reverse motions causes the inclined plane to push the nozzle-holding part transversely to said first geometrical axis, thereby moving the nozzles from the retracted position to the advanced position. By using an inclined plane for transferring the motion of the actuator part, it is possible to make a space saving configuration of the actuator as a whole. For instance, the actuator part may be moved along the vertical extension of the medical processing device, without requiring any additional vertical space, and the inclined plane can effectively transfer the vertical motion to a horizontal motion of the nozzle-holding part, without requiring excessive horizontal space.

According to at least one exemplary embodiment, the system further comprises a control unit configured to send motion requests to the actuator for making the actuator move the nozzles between said retracted position and said advanced position. The control unit may receive input signals based on which it may send said motion requests to the actuator. Input signals may, in some examples, be in received from a user interface. The user interface may, for instance be a control panel on the medical processing device and/or a remotely located input device (such as a remote computer or phone). For example, the staff may after confirming that the removable coupling device is located at the docking location send an input signal from the user interface to the control unit, wherein the control unit will in its turn send a motion request to the actuator in order to make the actuator move the nozzles to their advanced positions. In some examples, the input signals may be automatically generated to the control unit. For instance, there may be provided means for detecting that the removable coupling device is positioned at said docking location, whereupon an input signal to the control unit is automatically triggered. The control unit may however, before sending the motion request, await further input signals, e.g. an input signal confirming that the door to the chamber of the medical processing device has been closed.

The control unit may include a microprocessor, microcontroller, programmable digital signal processor or another programmable device. The control unit may also, or instead, include an application specific integrated circuit, a programmable gate array or programmable array logic, a programmable logic device, or a digital signal processor. Where it includes a programmable device such as the microprocessor, microcontroller or programmable digital signal processor mentioned above, the processor may further include computer executable code that controls operation of the programmable device. The control unit is preferably configured for use with medical processing devices, and provided with electronic instructions to perform and control the advancement and retraction of the nozzles.

As mentioned above, there may be provided means for detecting the position of the removable coupling device. Thus, in a general sense, according to at least one exemplary embodiment, the system further comprises a position detecting device, wherein the position detecting device is configured to indicate that the removable coupling device is positioned at said docking location. In line with the previous explanations, such a position detecting device may, in at least some examples, be in operative communication with the control unit. Thus, the positon detecting device may, for example, send an input signal to the control unit, thereby confirming to the control unit that the removable coupling device is correctly positioned for receive the nozzles. However, in other examples, the position detecting device may additionally, or alternatively, provide information about the position of the removable coupling device to a user interface. Such information may be conveyed in any suitably way, such as visually, activating a light or presenting a message on a screen, and/or audibly, such as sounding an audible tone or voice message.

Any suitable position detecting device may be implemented in the system of this disclosure. For instance, the position detecting device may be magnet based. A first magnet on the removable coupling device may interact with a magnet in the medical processing device, such as in the docking unit, wherein when the magnets become aligned, a signal is triggered, e.g. by means of a switch. Another possibility is to configure the position detecting device to be based on mechanical interaction, e.g. when the removable coupling device arrives at the docking location, it mechanically engages a switch which triggers a signal (such as the input signal to the control unit or a signal to a user interface). Yet another possibility is to have a position detecting device which includes an optical sensor, which visually detects the presence of the removable coupling device.

From the above discussion it can be understood that according to at least one exemplary embodiment, the system may comprise a position detecting device in informational communication with the control unit, wherein the position detecting device is configured to detect that the removable coupling device is positioned at said docking location, wherein upon receipt from the position detecting device that the removable coupling device has become positioned at said docking location, the control unit is configured to send a motion request to the actuator to cause the nozzles to be moved from the retracted position to the advanced position so as to engage the fluid passages of the removable coupling device. Thus, the engagement process may advantageously be automated.

According to at least one exemplary embodiment, each fluid passage has an inlet side for receiving a respective nozzle and an outlet side for connecting the channels of the medical article, in particular wherein the inlet side of each fluid passage includes a cavity for receiving a respective nozzle. Thus, the removable coupling device may be made relatively compact, with one side facing the nozzles and an opposite side facing towards the center of the chamber of the medical processing device. The fluid passages may suitably be straight through passages, and at least a part of those through passages are formed as nozzle-receiving cavities. Thus, it should be understood that the cross-section of the fluid passage may vary from the inlet side to the outlet side.

According to at least one exemplary embodiment, the removable coupling device comprises a plurality of connecting nipples defining the outlet sides of the flow passages, wherein the connecting nipples are configured to be connected to said channels of the medical article either directly or via connecting tubes interconnecting said channels with the connecting nipples. The connecting nipples may suitably be of standard dimensions which fit with existing connecting tubes and/or channels. Thus, the connecting nipples may suitably have dimensions which correspond to those of connectors in existing medical processing devices, such as washers-disinfectors.

As already mentioned above, in at least some exemplary embodiments, the removable coupling device may be configured to be removably mountable to a load carrier for carrying medical articles, such that insertion of the load carrier into the medical processing device causes the mounted removable coupling device to become positioned at said docking location. By having a pre-defined mounting position on the load carrier, the fluid passages of the removable coupling device may automatically become correctly aligned with the nozzles when the load carrier is inserted into the medical processing device. The load carrier may suitably be adapted to the dimension of the chamber of the medical processing device such that it is always inserted to the same position. For instance, the outer dimension of the load carrier may suitably substantially correspond to the inner dimension of the chamber, including any appropriate play. According to at least some exemplary embodiments the system may comprise one or more load carriers configured to receive a removable coupling device.

According to at least one exemplary embodiment, the removable coupling device is configured to be at least partly movable when mounted to the load carrier, and each nozzle is provided with a tapered tip portion for enabling automatic fine-adjustment of the position of the removable coupling device, said fine-adjustment being enabled by allowing the tip portions of the nozzles to enter the fluid passages even if the nozzles and the fluid passages are not perfectly coaxially aligned when the nozzles are moved from the retracted position. Thus, the removable coupling device may advantageously be "free floating", or have at least a small degree of freedom to move, when mounted on the load carrier. This configuration enables a self-centering docking of the removable coupling device. The removable coupling device may, within certain limits, be freely movable on the load carrier. The tapered tip portions, for example conical tip portions, of the nozzles allows the removable coupling device to become automatically correctly aligned when docked by movement of the nozzles from the retracted position to the advanced position. Hereby, correct alignment and sealing between the nozzles and the removable coupling device with its fluid passages may be achieved.

From the above discussion and the various examples presented, it should now be understood that in at least some exemplary embodiments, the removable coupling device is configured for connection to an endoscope, and the medical processing device comprises one of an endoscope washer-disinfector or an endoscope drying device.

According to at least one exemplary embodiment, the medical processing device is a first medical processing device, such as an endoscope washer-disinfector (EWD), the system further comprising a second medical processing device, such as a drying and storage cabinet (DSC), the second medical processing device being configured to receive medical articles to be subjected to a process medium,
the second medical processing device comprising a plurality of nozzles, each nozzle being movable between a retracted position and an advanced position,
the system further comprising a transporting arrangement,
wherein, upon determination by the control unit that the first medical processing device has completed the processing of the medical article, then the control unit is configured to control the transporting arrangement to transfer the removable coupling device and the medical article connected thereto, from the first medical processing device and into the second medical processing device for enabling the nozzles of the second medical processing device to become engaged with the fluid passages of the removable coupling device for further processing of the medical article in the second medical processing device.

This is beneficial as the medical article can stay connected to the removable coupling device throughout all the process steps, even when being moved from one medical processing device to another. This configuration is time-saving as there is no need to disconnect and reconnect all the channels of the medical article.

According to at least a second aspect of the present inventive concept, there is provided a method for treating a medical article having multiple channels, the method comprising:
- providing a removable coupling device comprising a plurality of fluid passages,
- connecting each channel of the medical article to a respective fluid passage,
- inserting the removable coupling device with the connected medical article into a medical processing device which is configured to receive medical articles to be subjected to a process medium,
- moving a plurality of nozzles of the medical processing device from a retracted position to an advanced position so that at least some of said nozzles, in particular all of said nozzles, become engaged with respective fluid passages of the removable coupling device, thereby enabling the medical processing device to supply the process medium from the nozzles, via the engaged fluid passages of the removable coupling device, into any channel of the medical article connected to the removable coupling device.

According to at least one exemplary embodiment of the method, said medical processing device is a first medical processing device, such as an endoscope washer-disinfector (EWD) the method further comprising:
- upon determination that the first medical processing device has completed the processing of the medical article, transferring the removable coupling device and the medical article connected thereto, from the first medical processing device and into a second medical processing device, such as a drying and storage cabinet (DSC),
- moving nozzles of the second medical processing device from a retracted position to an advanced position so that at least some of said nozzles of the second medical processing device, in particular all of said nozzles, become engaged with respective fluid passages of the removable coupling device, thereby enabling the second medical processing device to supply process medium from its nozzles, via the engaged fluid passages of the removable coupling device, into any channel of the medical article connected to the removable coupling device.

The reader will understand that the various features and exemplary embodiments discussed in connection with the system of the first aspect may be readily implemented as corresponding exemplary embodiments of the method of the second aspect.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. Further features of, and advantages with, the present invention will become apparent when studying the appended claims and the following description. The skilled person realizes that different features of the present invention may be combined to create embodiments other than those described in the following, without departing from the scope of the present invention as defined in the appended set of claims.

### BRIEF DESCRIPTION OF THE DRAWINGS

Fig. 1 illustrates, in accordance with at least one exemplary embodiment of the present disclosure, a system for supplying a process medium to medical articles having multiple channels.
Fig. 2 illustrates an example of a removable coupling device that may be used in various exemplary embodiments of the system of the present disclosure.
Fig. 3 illustrates the removable coupling device of Fig. 2 with connecting tubes attached to nipples of the removable coupling device.
Fig. 4 illustrates the removable coupling device connected to channels of a medical article, the removable coupling device being positioned at a docking location.
Figs. 5 and 6 are exemplary illustrations of retracted and advanced positions, respectively, of nozzles of a medical processing device and how the nozzles become engaged with fluid passages of a removable coupling device.
Fig. 7 illustrates an example of a nozzle-moving actuator which may be implemented in exemplary embodiments of the system of the present disclosure.
Fig. 8 is a schematic illustration of an example of automating the movement of the nozzles from the retracted positions to the advanced positions.

### DETAILED DESCRIPTION

The present disclosure will now be described more fully hereinafter with reference to the accompanying drawings, in which certain aspects of a system of the present disclosure are shown. The system may, however, be embodied in many different forms and should not be construed as limited to the embodiments and aspects set forth herein; rather, the embodiments are provided by way of example so that this disclosure will be thorough and complete, and will fully convey the scope of the system to those skilled in the art. Accordingly, it is to be understood that the present disclosure is not limited to the embodiments described herein and illustrated in the drawings; rather, the skilled person will recognize that many changes and modifications may be made within the scope of the appended claims. Like reference numerals refer to like elements throughout the description.

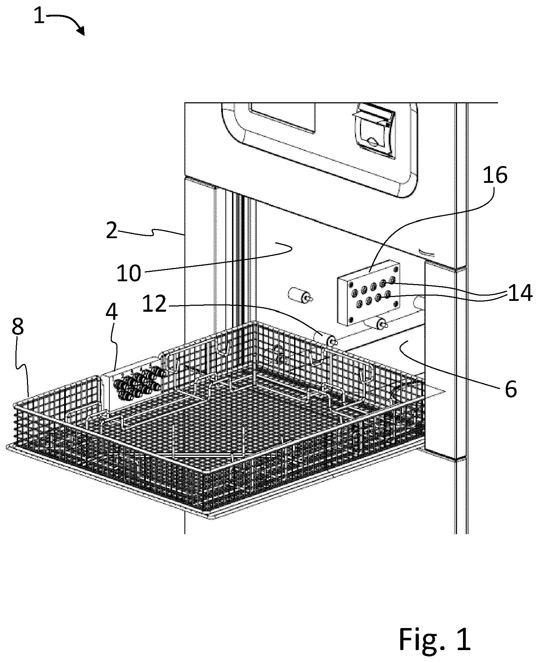

Fig. 1 illustrates, in accordance with at least one exemplary embodiment of the present disclosure, a system 1 for supplying a process medium to medical articles having multiple channels. The system 1 comprises a medical processing device 2 and a removable coupling device 4. The medical processing device 2 is configured to receive medical articles to be subjected to a process medium. The medical processing device comprises an internal chamber 6 in which the medical articles are located when being subjected to the process medium.

As illustrated in Fig. 1, there may be provided with a load carrier 8 for holding one or more medical articles. When a medical article is held in the load carrier 8, the load carrier 8 may be inserted into the internal chamber 6 for processing of the medical article. The load carrier 8 is here illustrated in the form of a basket. However, it should be understood that a basket is just one example of a load carrier, i.e. a structure for holding a medical article. Some other examples of such structures or load carriers that may be used in connection with the system 1 of this disclosure are bins, boxes, trays or platforms.

As illustrated in Fig. 1, an inner side wall 10, which partly defines the internal chamber 6 of the medical processing device 2 may be provided with a plurality of rollers 12 for guiding the load carrier 8 or a different structure upon entry into the internal chamber 6.

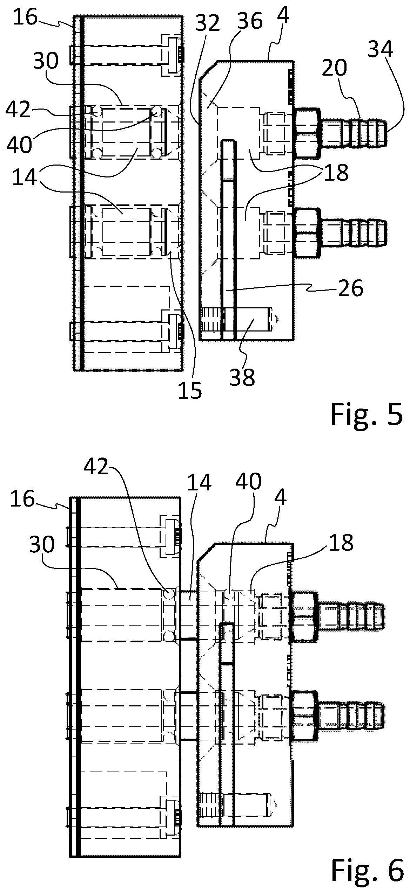

The medical processing device 2 may for example be a washer-disinfector or a drying device, in particular an endoscope washer-disinfector or an endoscope drying device. The medical processing device 2 may thus be used for washing (cleaning) and/or disinfecting, or for drying medical articles. The medical processing device 2 comprises a plurality of nozzles 14, each nozzle 14 being movable between a retracted position and an advanced position (this will be discussed in more detail in relation to Figs. 5 and 6). In Fig. 1, the nozzles 14 are illustrated as being provided in a docking unit 16 mounted to the inner side wall 10. However, in other exemplary embodiments, the movable nozzles 14 may be provided in other structures, e.g. in the actual side wall 10 without any specific docking unit 16 being present or visible. For example, the outer surface of the docking unit 16 might instead be flush with the inner wall 10 of the processing chamber.

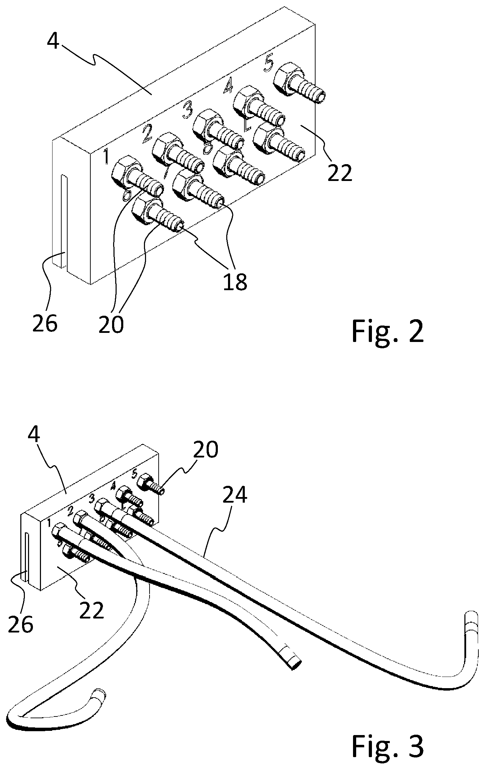

Turning now to Fig. 2, the removable coupling device 4 comprises a plurality of fluid passages 18. In this example, a part of each fluid passage 18 is defined by a respective connecting nipple 20 projecting from a support part 22 of the removable coupling device 4. Each fluid passage 18 may suitably extend from the end of the nipple 20, through the support part 22, and to the opposite side of the support part 22. The plurality of connecting nipples 20 may define the outlet sides of the flow passages 18, whereas the ends of the flow passages 18 on the opposite side of the support part 22 may define the inlet sides of the flow passages 18. The inlet sides of the flow passages 18 are configured to engage with the nozzles 14 which have previously been presented in Fig. 1. In Fig. 2 only the outlet side of each flow passages 18 is visible, while the inlet side is concealed in this view. The connecting nipples 20 may be configured to be connected to the channels of the medical article, either directly, or via connecting tubes that interconnect said channels with the connecting nipples 20. The latter option is illustrated in Fig. 3, which shows three connecting tubes 24 connected to a respective connecting nipple 20. It should be understood that the connecting nipples 20 are just one conceivable example for connecting the removable coupling device 4 to the channels of the medical article. In other examples, the connection may be achieved in different manners. For instance, in some examples, a support part 22 may have a different thickness and present integrated recesses for receiving channels and/or connecting tubes 24.

In both Fig. 2 and Fig. 3, there is illustrated a slit 26 extending from a lower end of the removable coupling device 4, in particular from the lower end of the support part 22 of the removable coupling device 4. This enables a simple mounting of the removable coupling device 4 onto a load carrier, such as the illustrated basket in Fig. 1. However, other mounting features may be used instead of, or in addition to, the slit 26. For instance, the removable coupling device 4 may, in some examples, be mounted by means of one or more clamps, by screw joints, by magnets, etc. In any one of these examples, the mounting may suitably be made in a removable way.

From the above it should thus be understood that the removable coupling device 4 may not only be removed from the medical processing device 2, but also from the load carrier 8. However, in some exemplary embodiments, the removable coupling device 4 may be integrated with the load carrier 8, but still be removable from the medical processing device 2 for allowing convenient connection to the channels of a medical article outside of the medical processing device 2. Thus, in a general sense, the removable coupling device 4 may comprise or may be part of a structure for holding the medical article when the medical article is connected to the removable coupling device 4. Regardless of the specific configuration of the removable coupling device 4, being removable from a load carrier 8 or not, the removable coupling device 4 is suitably configured for connection to an endoscope, and the medical processing device 2 may comprise one of an endoscope washer-disinfector or an endoscope drying device.

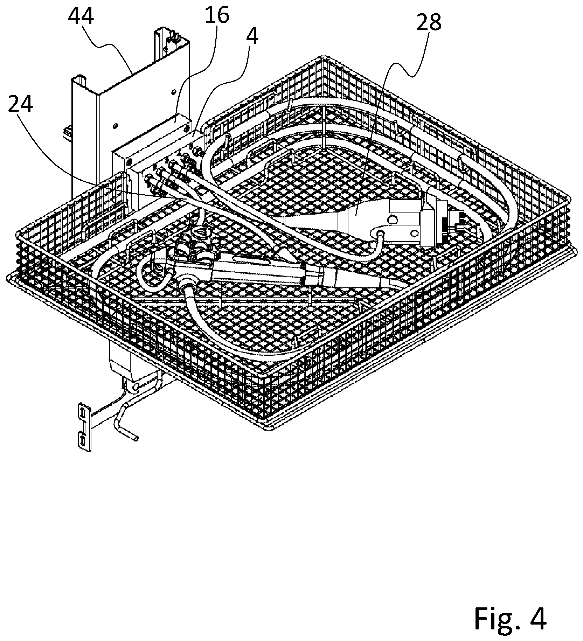

The removable coupling device 4 is configured to be provided at a docking location inside the medical processing device 2 so that movement of the nozzles 14 from the retracted position to the advanced position causes at least some of the nozzles 14, in particular all of said nozzles 14 to become engaged with respective fluid passages 18 of the removable coupling device 4. Such position of the removable coupling device 4 is illustrated in Fig. 4. Thus, Fig. 4 shows the removable coupling device 4 being positioned at its docking location. Thus, in this state the removable coupling device 4 has been inserted into the internal chamber 6 of the medical processing device 2 (the internal chamber 6 in Fig. 1 is not shown in Fig. 4). As can be seen in Fig. 4, the docking location is aligned with the docking unit 16 such that, when the removable coupling device 4 is positioned at the docking location, each fluid passage is co-axially aligned with a respective nozzle.

In Fig. 4 there is illustrated a medical article 28 having multiple channels. In this example the medical article 28 is illustrated as an endoscope. The connecting tubes 24 extend from the removable coupling device 4 to a respective channel of the medical article 28. Hereby, each one of the three connected channels of the medical article 28 can become flushed. In particular, upon engagement of the nozzles with the fluid passages of the removable coupling device 4, the medical processing device will be able to supply the process medium from the nozzles, via the engaged fluid passages of the removable coupling device 4, into the channels of the medical article 28 connected to the removable coupling device 4.

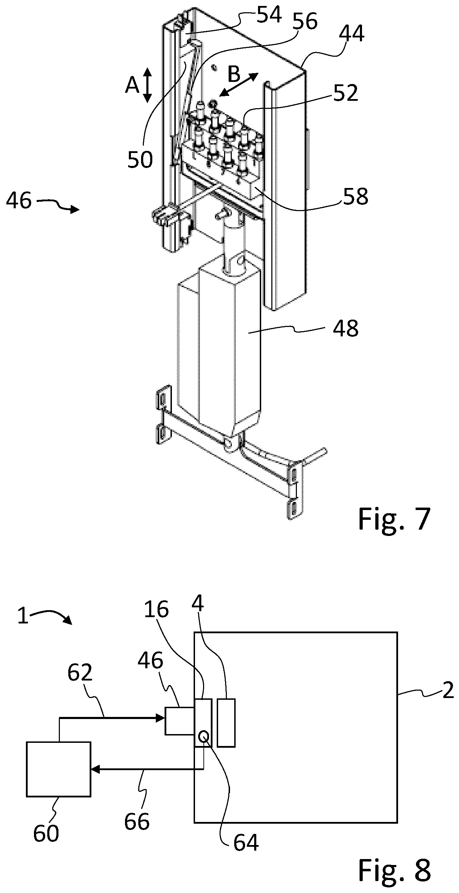

Fig. 4 illustrates that the docking unit 16 is mounted to a bracket 44. On the opposite side of the bracket 44, there may be provided an actuator (not visible in Fig. 4) which is configured to move the nozzles between said retracted position and said advanced position. An example of such an actuator will be discussed later in relation to Fig. 7.

The movement of the nozzles 14 will now be discussed in more detail with respect to Figs. 5 and 6, which are exemplary illustrations of retracted and advanced positions, respectively, of nozzles 14 of a medical processing device and how the nozzles 14 become engaged with fluid passages 18 of a removable coupling device 4.

Starting with Fig. 5, a lateral partly cross-sectional view illustrates a state in which the removable coupling device 4 has arrived at its docking location. In this position, the fluid passages 18 of the removable coupling device 4 are aligned with, but not yet engaged with, the nozzles 14 of the medical processing device. More specifically, now that the removable coupling device 4 is positioned at the docking location, each fluid passage 18 is co-axially aligned with a respective nozzle 14.

Whether the nozzles 14 are provided in a docking unit or in a sidewall of the medical processing device, there may suitably be a plurality of compartments 30 for housing the nozzles 14. Docking units 16 having an outer surface which is flush or continuous with the adjacent wall surface are still considered to be docking units 16 herein. Fig. 5 may, for instance, illustrate a docking unit 16 comprising a plurality of compartments 30, one for each nozzle 14. In the illustrated retracted position in Fig. 5, each nozzle 14 is located at least partially inside its respective compartment 30, in particular fully inside its respective compartment 30.

In the removable coupling device 4, each fluid passage has an inlet side 32 for receiving a respective nozzle 14 and an outlet side 34 for connecting to the channels of the medical article. As illustrated in Fig. 5, the inlet side 32 of each fluid passage 18 may include a cavity 36 for receiving a respective nozzle 14. As previously discussed, the outlet side 34 of each fluid passage 18 may be defined by a respective connecting nipple 20.

As mentioned earlier, there may be various mounting features for mounting the removable coupling device 4 to a basket or any other suitable type of load carrier. Fig. 5 illustrates that in addition to a slit 26, there may be provided a treaded bore 38 configured to receive a threaded fastening element, such as a bolt, to temporarily tighten the removable coupling device 4 to the load carrier.

Turning now to Fig. 6, illustrating the advance position of the nozzles 14, each nozzle 14 now projects out from its respective compartment 30. Each nozzle also advances and projects out beyond the adjacent surface of the docking unit 16 in the advances position. Furthermore, each nozzle 14 has now become engaged with a respective fluid passage 18 of the removable coupling device 4. As illustrated in the example of Fig. 6, in the advanced position of the nozzles 14, the nozzles 14 may be configured to project into the fluid passages 18 of the removable coupling device 4. Thus, the nozzles 14 provide male parts and the fluid passages 18 provide female parts of the engagement. However, it should be understood that the reverse may be realized in other exemplary embodiments, i.e. the nozzles forming female parts and the fluid passages forming male parts.

As can be seen in Figs. 5 and 6, each nozzle 14 may be provided with at least one seal 40, 42 configured to follow the movement of the nozzle 14. The seals could be made of rubber, plastic, or another resilient material. In the illustrated example, each nozzle comprises two seals 40, 42, namely a front seal 40 and a rear seal 42. Each seal 40, 42 has an inner periphery that is in encircling contact with the nozzle 14. As illustrated in Fig. 6, when the nozzles 14 are in the advanced position and are engaged with respective fluid passages 18 of the removable coupling device 4, an outer periphery of each front seal 40 seals against a circumferential wall portion of the engaged fluid passage 18. An outer periphery of each rear seal 42 seals against a circumferential wall portion of the compartment 30 from which the nozzle 14 projects. The front seals 40 counteract backwards leakage around the external surfaces of the nozzles 14. The rear seals 42 counteract leakage of process medium present in the internal chamber of the medical processing device from leaking back through the compartments 30 around the nozzles 14. In this connection, it may be pointed out that the medical processing chamber may suitably have other nozzles or outlets than those illustrated in the figures. Thus, in addition to the herein illustrated nozzles 14 used for providing process medium to internal volumes of medical articles, in particular into multiple channels of medical articles, the medical processing device may also be provided with additional nozzles or other types of outlets, for providing process medium externally of the medical articles. Such additional nozzles or outlets may, for instance, be provided on one or more spray wings, which may, for instance, be mounted to a side wall, a bottom wall or a top wall of the medical processing device.

As can be seen in Fig. 6, the front seals 40 of the nozzles 14 provide a tight engagement with the walls of the fluid passages 18. Because of the engagement between a plurality of nozzles 14 and a plurality of fluid passages 18, the degrees of freedom are limited. In particular, in the advanced position of the nozzles 14, when they have engaged the fluid passages 18, the nozzles 14 prevent the removable coupling device 4 from moving in a radial direction relative to the axial extension of any one of the nozzles 14.

The removable coupling device 4 may be allowed to be slightly movable when mounted to the load carrier. Fine-adjustment of the removable coupling device 4 may be achieved by designing each nozzle 14 with tapered tip portion 15 (see Fig. 5). Even if the removable coupling device 4 with its fluid passages 18 is not completely aligned with the nozzles 14, the tapered tip portions 15 will be allowed to enter the fluid passages during movement from the retracted position to the advanced position. Upon continued movement of the nozzles 14 towards the advanced position, any minor offset of the removable coupling device 4 will automatically be adjusted and the removable coupling device 4 will become correctly aligned with the docking unit 16.

As illustrated in Figs. 1, 5 and 6 the nozzles 14 may be distributed to form one or more rows of nozzles 14. In these examples, two rows of nozzles 14 are shown. In other exemplary embodiments, there may be provided any other suitable number of rows, or number of nozzles 14, than what has been illustrated. Each nozzle 14 may advance along a different respective central axis out and away from a side wall 10 or docking unit 16 of the medical processing device 2 when moving into the advanced position. Said central axes may be mutually parallel.

As mentioned previously in relation to Fig. 4, the docking unit 16 may be mounted to a bracket 44, and there may be provided an actuator on the other side of the bracket 44. An example of such an actuator is illustrated in Fig. 7.

Thus, Fig. 7 illustrates an actuator 46 located on the other side of the bracket 44 shown in Fig. 4. The actuator 46 comprises a motor 48 and a movable actuator part 50. Before going into detail of the movement patterns of the different components, it should be pointed out that the plurality of nozzle-like elements 52 visible in Fig. 7 are not the same nozzles 14 as discussed with respect to the previous drawing figures. These elements 52 are connectors for connecting conduits to one or more pumps supplying the process medium from a source. The elements 52 are in fluid communication with the nozzles 14, but the nozzles 14 are not visible in this view, but extend horizontally on the other side of the bracket 44.

In this example, the movable actuator part 50 is in the form of a carriage which can be moved up and down along a track 54 in the bracket 44. It is the motor 48 that is configured to drive the movable actuator part 50 alternatingly in a forward and reverse motion along a first geometrical axis. In this illustration, the first geometrical axis is a vertical axis. Accordingly, in this illustration, the motor 48 is configured to drive the movable actuator part 50 alternatingly upwards and downwards, as indicated by double-arrow A. One of said forward and reverse motions of the movable actuator part 50 causes the nozzles to move transversely to said first geometrical axis from the retracted position to the advanced position, and the other one of said forward and reverse motions of the movable actuator part 50 causes the nozzles to return from the advanced position to the retracted position. Thus, in this example, the transverse movement will be a horizontal movement, as indicated by double-arrow B.

As illustrated in this example, the conversion of the vertical motion of the movable actuator part 50 to a horizontal movement of the nozzles 14 can be achieved by providing the movable actuator part 50 with an inclined plane, such as an inclined rib or ridge 56 or the like. When the motor 48 pulls the actuator part 50 down along the track 54, the inclined ridge 56 will push a nozzle-holding part, such as a block 58 holding the elements 52 as well as the nozzles 14. More specifically, because the block 58 is prevented from moving vertically, the pushing by the inclined ridge 56 will cause the block 58 to move horizontally. The block 58 holds the nozzles 14 which will thus follow the motion of the block 58, resulting in a movement from the retracted position to the advanced position of the nozzles 14. The block 58 may be spring-biased such that when the motor 48 raises the movable actuator part 50 along the track 54, a spring (not illustrated) returns the block 58, thereby also returning the nozzles 14 to the retracted position.

It should be understood that the illustrated actuator 46 is just one example, and that the medical processing device may be equipped with other types of actuators, motors, etc. for effecting a positional change of the nozzles between said retracted position and said advanced position.

Fig. 8 is a schematic illustration of an example of automating the movement of the nozzles from the retracted positions to the advanced positions. Fig. 8 illustrates that the system 1 may comprise a control unit 60 configured to send motion requests 62 to an actuator 46 (such as the one exemplified in Fig. 7 or any other suitable actuator), for making the actuator 46 move the nozzles between said retracted position and said advanced position.

As illustrated in Fig. 8, the system 1 may further comprise a position detecting device 64. The position detecting device 64 may be configured to indicate that the removable coupling device 4 is positioned at said docking location within the medical processing device 2. In other words, the position detecting device 64 may be configured to indicate that the removable coupling device 4 is in position for its fluid passages to become engaged with the nozzles. As previously discussed in this disclosure, some examples of position detecting devices 4 that may be used in the system 1 are magnetic sensors, mechanical switches, optical sensors etc.

The position detecting device 64 may be in informational communication with the control unit 60. Thus, when the position detecting device 64 has detected that the removable coupling device 4 is positioned at the docking location (e.g. as discussed previously, positioned at the docking unit 16), the control unit 60 may receive a message 66 from the position detecting device 64. The message 66 may for example be an analogue signal, such as a voltage signal, or it may be a digital signal such as containing digital data. The control unit 60 may be configured to send the motion request 62 to the actuator 46 when it has received information from the position detecting device 64 that the removable coupling device 4 has become positioned at said docking location. The motion request 62 (e.g. an analogue or digital signal) may thus control the actuator 46 to cause the nozzles to be moved from the retracted position to the advanced position so as to engage the fluid passages of the removable coupling device 4.

This disclosure includes systems for supplying a process medium to medical articles, and methods of operating such systems. In particular, this disclosure includes systems and methods for enabling time-efficient processing, such as washing, cleaning, disinfecting and/or drying, of multiple internal channels of medical articles. This disclosure includes docking systems, endoscope processing machinery, and extended systems including mutually compatible endoscope washer-disinfectors, endoscope drying and/or storage cabinets, and coupling devices 4 which are compatible with both the washer-disinfector(s) and the drying/storage cabinet(s) for efficient transfers there between. It should be understood that various features disclosed herein are contemplated and disclosed in their various combinations and sub-combinations.

## Claims

1. A system (1) for supplying a process medium to medical articles (28) having multiple channels, the system (1) comprising:
- a medical processing device (2) configured to receive medical articles (28) to be subjected to a process medium, the medical processing device (2) comprising a plurality of nozzles (14), each nozzle (14) being movable between a retracted position and an advanced position,
- a removable coupling device (4) comprising a plurality of fluid passages (18), each fluid passage (18) being configured to be connectable to a respective channel of a medical article (28) having multiple channels before the removable coupling device (4) is inserted into the medical processing device (2),
wherein the removable coupling device (4) is configured to be positioned at a docking location inside the medical processing device (2) so that movement of the nozzles (14) from said retracted position to said advanced position causes at least some of said nozzles (14), in particular all of said nozzles (14), to become engaged with respective fluid passages (18) of the removable coupling device (4), thereby enabling the medical processing device (2) to supply the process medium from the nozzles (14), via the engaged fluid passages (18) of the removable coupling device (4), into any channel of the medical article (28) connected to the removable coupling device (4),
wherein the medical processing device (2) comprises an actuator (46), wherein the actuator is configured to move the nozzles (14) between said retracted position and said advanced position,
wherein the actuator (46) comprises a motor (48) and a movable actuator part (50), wherein the motor (48) is configured to drive the movable actuator part (50) alternatingly in a forward and reverse motion along a first geometrical axis, **characterized in that** one of said forward and reverse motions of the movable actuator part (50) causes the nozzles (14) to move transversely to said first geometrical axis from the retracted position to the advanced position, and
the other one of said forward and reverse motions of the movable actuator part (50) causes the nozzles (14) to return from the advanced position to the retracted position.

2. The system (1) according to claim 1, wherein the medical processing device (2) comprises a docking unit (16), the docking unit (16) comprising a plurality of compartments (30), one for each nozzle (14), wherein
- in said retracted position, each nozzle (14) is located at least partially inside its respective compartment (30), in particular fully inside its respective compartment (30), and
- in said advanced position, each nozzle (14) projects out from its respective compartment (30).

3. The system (1) according to claim 2, wherein said docking location for the removable coupling device (4) is aligned with the docking unit (16) such that, when the removable coupling device (4) is positioned at the docking location, each fluid passage (18) is co-axially aligned with a respective nozzle (14).

4. The system (1) according to any one of claims 1-3, wherein in the advanced position of the nozzles (14), the nozzles (14) are configured to project into the fluid passages (18) of the removable coupling device (4).

5. The system (1) according to any one of claims 1-4, wherein each nozzle (14) is provided with at least one seal (40, 42) configured to follow the movement of the nozzle (14).

6. The system (1) according to claim 2 or any one of claims 3-5 when dependent on claim 2, wherein each nozzle (14) comprises a front seal (40) and a rear seal (42), each having an inner periphery that is in encircling contact with the nozzle (14), wherein when the nozzle (14) is in the advanced position and is engaged with a fluid passage (18), an outer periphery of the front seal (40) seals against a circumferential wall portion of the engaged fluid passage (18), and an outer periphery of the rear seal (42) seals against a circumferential wall portion of the compartment from which the nozzle (14) projects.

7. The system (1) according to any one of claims 1-6, wherein in the advanced position of the nozzles (14), when they have engaged the fluid passages (18), the nozzles (14) prevent the removable coupling device (4) from moving in a radial direction relative to the axial extension of any one of the nozzles (14).

8. The system (1) according to any one of claims 1-7, wherein the actuator part (50) comprises an inclined plane configured to interact with a nozzle-holding part, wherein movement of the actuator part (50) in one of said forward and reverse motions causes the inclined plane to push the nozzle-holding part transversely to said first geometrical axis, thereby moving the nozzles (14) from the retracted position to the advanced position.

9. The system (1) according to any one of claims 1-8, further comprising a control unit (60) and a position detecting device (64) in informational communication with the control unit (60), wherein the position detecting device (64) is configured to indicate that the removable coupling device (4) is positioned at said docking location, wherein upon receipt of information from the position detecting device (64) that the removable coupling device (4) has become positioned at said docking location, the control unit (60) is configured to send a motion request (62) to the actuator to cause the nozzles (14) to be moved from the retracted position to the advanced position so as to engage the fluid passages (18) of the removable coupling device (4).

10. The system (1) according to any one of claims 1-9, wherein each fluid passage (18) has an inlet side (32) for receiving a respective nozzle (14) and an outlet side (34) for connecting to the channels of the medical article (28), in particular wherein the inlet side (32) of each fluid passage (18) includes a cavity (36) for receiving a respective nozzle (14).

11. The system (1) according to claim 10, wherein the removable coupling device (4) comprises a plurality of connecting nipples (20) defining the outlet sides (34) of the flow passages (18), wherein the connecting nipples (20) are configured to be connected to said channels of the medical article (28) either directly or via connecting tubes (24) interconnecting said channels with the connecting nipples (20).

12. The system (1) according to any one of claims 1-11, wherein the removable coupling device (4) is configured to be removably mountable to a load carrier (8) for carrying medical articles (28), such that insertion of the load carrier (8) into the medical processing device (2) causes the mounted removable coupling device (4) to become positioned at said docking location.

13. The system (1) according to claim 12, wherein the removable coupling device (4) is configured to be at least partly movable when mounted to the load carrier (8), wherein each nozzle (14) is provided with a tapered tip portion for enabling fine-adjustment of the position of the removable coupling device (4), said fine-adjustment being enabled by allowing the tip portions of the nozzles (14) to enter the fluid passages (18) during advancement from the retracted position to the advanced position even if the nozzles (14) and the fluid passages are not coaxially aligned when the nozzles (14) are moved out from the retracted position; in particular wherein the removable coupling device (4) is configured to be at least partly movable with respect to the load carrier (8).

14. The system (1) according to any previous claim, wherein the nozzles (14) each advance along a different respective central axis out and away from a side wall (10) or docking unit (16) of the medical processing device (2) when moving into the advanced position, in particular wherein said respective central axes are mutually parallel.

15. The system (1) according to any previous claim, wherein the removable coupling device (4) comprises or is part of a structure for holding the medical article (28) when the medical article (28) is connected to the removable coupling device (4), in particular wherein the structure for holding the medical article (28) comprises a basket, bin, box, tray, or platform.

16. The system (1) according to any previous claim, wherein the removable coupling device (4) is configured for connection to an endoscope, and the medical processing device (2) comprises one of an endoscope washer-disinfector or an endoscope drying device.

## Patentansprüche

1. System (1) zur Zufuhr eines Prozessmediums zu medizinischen Artikeln (28) mit mehreren Kanälen, wobei das System (1) Folgendes umfasst:
- eine medizinische Verarbeitungsvorrichtung (2), die dazu konfiguriert ist, einem Prozessmedium zu unterziehende medizinische Artikel (28) aufzunehmen, wobei die medizinische Verarbeitungsvorrichtung (2) eine Vielzahl von Düsen (14) umfasst, wobei jede Düse (14) zwischen einer eingezogenen und einer vorgeschobenen Position bewegbar ist,
- eine entfernbare Kopplungsvorrichtung (4), die eine Vielzahl von Fluiddurchlässen (18) umfasst, wobei jeder Fluiddurchlass (18) dazu konfiguriert ist, mit einem jeweiligen Kanal eines medizinischen Artikels (28) mit mehreren Kanälen verbindbar zu sein, bevor die entfernbare Kopplungsvorrichtung (4) in die medizinische Verarbeitungsvorrichtung (2) eingeführt wird,
wobei die entfernbare Kopplungsvorrichtung (4) dazu konfiguriert ist, an einer Verankerungsstelle innerhalb der medizinischen Verarbeitungsvorrichtung (2) so positioniert zu werden, dass eine Bewegung der Düsen (14) aus der eingezogenen Position in die vorgeschobene Position bewirkt, dass mindestens einige der Düsen (14), insbesondere alle Düsen (14), mit den jeweiligen Fluiddurchlässen (18) der entfernbaren Kopplungsvorrichtung (4) in Eingriff gelangen, wodurch der medizinischen Verarbeitungsvorrichtung (2) ermöglicht wird, das Prozessmedium aus den Düsen (14) über die in Eingriff stehenden Fluiddurchlässe (18) der entfernbaren Kopplungsvorrichtung (4) in einen beliebigen Kanal des medizinischen Artikels (28), der mit der entfernbaren Kopplungsvorrichtung (4) verbunden ist, zuzuführen,
wobei die medizinische Verarbeitungsvorrichtung (2) einen Aktor (46) umfasst, wobei der Aktor dazu konfiguriert ist, die Düsen (14) zwischen der eingezogenen Position und der vorgeschobenen Position zu bewegen,
wobei der Aktor (46) einen Motor (48) und ein bewegbares Aktorteil (50) umfasst,
wobei der Motor (48) dazu konfiguriert ist, das bewegbare Aktorteil (50) abwechselnd in einer Vorwärts- und einer Rückwärtsbewegung entlang einer ersten geometrischen Achse anzutreiben, **dadurch gekennzeichnet, dass** eine der Vorwärts- und der Rückwärtsbewegung des bewegbaren Aktorteils (50) bewirkt, dass sich die Düsen (14) quer zu der ersten geometrischen Achse aus der eingezogenen Position in die vorgeschobene Position bewegen, und die andere der Vorwärts- und der Rückwärtsbewegung des bewegbaren Aktorteils (50) bewirkt, dass die Düsen (14) aus der vorgeschobenen Position in die eingezogene Position zurückkehren.

2. System (1) nach Anspruch 1, wobei die medizinische Verarbeitungsvorrichtung (2) eine Verankerungseinheit (16) umfasst, wobei die Verankerungseinheit (16) eine Vielzahl von Kammern (30), eine für jede Düse (14), umfasst, wobei
- sich jede Düse (14) in der eingezogenen Position mindestens teilweise innerhalb ihrer jeweiligen Kammer (30), insbesondere vollständig innerhalb ihrer jeweiligen Kammer (30), befindet und
- jede Düse (14) in der vorgeschobenen Position aus ihrer jeweiligen Kammer (30) hervorsteht.

3. System (1) nach Anspruch 2, wobei die Verankerungsstelle für die entfernbare Kopplungsvorrichtung (4) mit der Verankerungseinheit (16) derart ausgerichtet ist, dass, wenn die entfernbare Kopplungsvorrichtung (4) an der Verankerungsstelle positioniert ist, jeder Fluiddurchlass (18) koaxial mit einer jeweiligen Düse (14) ausgerichtet ist.

4. System (1) nach einem der Ansprüche 1-3, wobei die Düsen (14) in der vorgeschobenen Position der Düsen (14) dazu konfiguriert sind, in die Fluiddurchlässe (18) der entfernbaren Kopplungsvorrichtung (4) vorzustehen.

5. System (1) nach einem der Ansprüche 1-4, wobei jede Düse (14) mit mindestens einer Dichtung (40, 42) versehen ist, die dazu konfiguriert ist, der Bewegung der Düse (14) zu folgen.

6. System (1) nach Anspruch 2 oder nach einem der Ansprüche 3-5, wenn abhängig von Anspruch 2, wobei jede Düse (14) eine vordere Dichtung (40) und eine hintere Dichtung (42) umfasst, die jeweils einen Innenumfang aufweisen, der in umlaufendem Kontakt mit der Düse (14) steht, wobei, wenn sich die Düse (14) in der vorgeschobenen Position befindet und mit einem Fluiddurchlass (18) in Eingriff steht, ein Außenumfang der vorderen Dichtung (40) gegen einen in Umfangsrichtung verlaufenden Wandabschnitt des in Eingriff stehenden Fluiddurchlasses (18) abdichtet und ein Außenumfang der hinteren Dichtung (42) gegen einen in Umfangsrichtung verlaufenden Wandabschnitt der Kammer, aus der die Düse (14) hervorsteht, abdichtet.

7. System (1) nach einem der Ansprüche 1-6, wobei die Düsen (14) in der vorgeschobenen Position der Düsen (14), wenn sie die Fluiddurchlässe (18) in Eingriff genommen haben, die entfernbare Kopplungsvorrichtung (4) daran hindern, sich in einer radialen Richtung relativ zu der axialen Erstreckung einer beliebigen der Düsen (14) zu bewegen.

8. System (1) nach einem der Ansprüche 1-7, wobei das Aktorteil (50) eine geneigte Ebene umfasst, die dazu konfiguriert ist, mit einem düsenhaltenden Teil zu interagieren, wobei eine Bewegung des Aktorteils (50) in einer der Vorwärts- und der Rückwärtsbewegung bewirkt, dass die geneigte Ebene das düsenhaltende Teil quer zu der ersten geometrischen Ebene drückt, wodurch die Düsen (14) aus der eingezogenen Position in die vorgeschobene Position bewegt werden.

9. System (1) nach einem der Ansprüche 1-8, ferner umfassend eine Steuereinheit (60) und eine Positionsdetektionsvorrichtung (64) in Informationskommunikation mit der Steuereinheit (60), wobei die Positionsdetektionsvorrichtung (64) dazu konfiguriert ist, anzugeben, dass die entfernbare Kopplungsvorrichtung (4) an der Verankerungsstelle positioniert ist, wobei bei Empfang von Informationen von der Positionsdetektionsvorrichtung (64), dass die entfernbare Kopplungsvorrichtung (4) an der Verankerungsstelle positioniert wurde, die Steuereinheit (60) dazu konfiguriert ist, eine Bewegungsanforderung (62) an den Aktor zu senden, um zu bewirken, dass die Düsen (14) aus der eingezogenen Position in die vorgeschobene Position bewegt werden, um die Fluiddurchlässe (18) der entfernbaren Kopplungsvorrichtung (4) in Eingriff zu nehmen.

10. System (1) nach einem der Ansprüche 1-9, wobei jeder Fluiddurchlass (18) eine Einlassseite (32) zum Aufnehmen einer jeweiligen Düse (14) und eine Auslassseite (34) zum Verbinden der Kanäle des medizinischen Artikels (28) aufweist, wobei insbesondere die Einlassseite (32) jedes Fluiddurchlasses (18) einen Hohlraum (36) zum Aufnehmen einer jeweiligen Düse (14) beinhaltet.

11. System (1) nach Anspruch 10, wobei die entfernbare Kopplungsvorrichtung (4) eine Vielzahl von Verbindungsnippeln (20) umfasst, die die Auslassseiten (34) der Strömungsdurchlässe (18) definieren, wobei die Verbindungsnippel (20) dazu konfiguriert sind, mit den Kanälen des medizinischen Artikels (28) entweder direkt oder über Verbindungsschläuche (24), die die Kanäle mit den Verbindungsnippeln (20) verbinden, verbunden zu sein.

12. System (1) nach einem der Ansprüche 1-11, wobei die entfernbare Kopplungsvorrichtung (4) dazu konfiguriert ist, entfernbar an einem Lastträger (8) zum Tragen von medizinischen Artikeln (28) derart montierbar zu sein, dass eine Einführung des Lastträgers (8) in die medizinische Verarbeitungsvorrichtung (2) bewirkt, dass die montierte entfernbare Kopplungsvorrichtung (4) an der Verankerungsstelle positioniert wird.

13. System (1) nach Anspruch 12, wobei die entfernbare Kopplungsvorrichtung (4) dazu konfiguriert ist, mindestens teilweise bewegbar zu sein, wenn sie an dem Lastträger (8) montiert ist, wobei jede Düse (14) mit einer konischen Spitze zum Ermöglichen einer Feineinstellung der Position der entfernbaren Kopplungsvorrichtung (4) versehen ist, wobei die Feineinstellung ermöglicht wird, indem zugelassen wird, dass die Spitzenabschnitte der Düsen (14) in die Fluiddurchlässe (18) während des Vorschubs aus der eingezogenen Position in die vorgeschobene Position eintreten, auch wenn die Düsen (14) und die Fluiddurchlässe nicht koaxial ausgerichtet sind, wenn die Düsen (14) aus der eingezogenen Position bewegt werden; wobei insbesondere die entfernbare Kopplungsvorrichtung (4) dazu konfiguriert ist, mindestens teilweise in Bezug auf den Lastträger (8) bewegbar zu sein.

14. System (1) nach einem der vorhergehenden Ansprüche, wobei die Düsen (14) jeweils entlang einer anderen jeweiligen Mittelachse heraus- und von einer Seitenwand (10) oder einer Verankerungseinheit (16) der medizinischen Verarbeitungsvorrichtung (2) weggeschoben werden, wenn sie in die vorgeschobene Position bewegt werden, wobei insbesondere die jeweiligen Mittelachsen zueinander parallel sind.

15. System (1) nach einem der vorhergehenden Ansprüche, wobei die entfernbare Kopplungsvorrichtung (4) eine Struktur zum Halten des medizinischen Artikels (28) umfasst oder ein Teil davon ist, wenn der medizinische Artikel (28) mit der entfernbaren Kopplungsvorrichtung (4) verbunden ist, wobei insbesondere die Struktur zum Halten des medizinischen Artikels (28) einen Korb, einen Eimer, einen Kasten, eine Schale oder eine Plattform umfasst.

16. System (1) nach einem der vorhergehenden Ansprüche, wobei die entfernbare Kopplungsvorrichtung (4) zur Verbindung mit einem Endoskop konfiguriert ist und die medizinische Verarbeitungsvorrichtung (2) eine von einer Endoskopreinigungs- und -desinfektionseinrichtung oder einer Endoskoptrocknungsvorrichtung umfasst.

## Revendications

1. Système (1) pour fournir un milieu de traitement à des articles médicaux (28) à canaux multiples, le système (1) comprenant :
- un dispositif de traitement médical (2) configuré pour recevoir des articles médicaux (28) devant être soumis à un milieu de traitement, le dispositif de traitement médical (2) comprenant une pluralité de buses (14), chaque buse (14) étant mobile entre une position rétractée et une position avancée,
- un dispositif de couplage amovible (4) comprenant une pluralité de passages de fluide (18), chaque passage de fluide (18) étant configuré pour pouvoir être raccordé à un canal respectif d'un article médical (28) à canaux multiples avant que le dispositif de couplage amovible (4) ne soit inséré dans le dispositif de traitement médical (2),
dans lequel le dispositif de couplage amovible (4) est configuré pour être positionné à un emplacement d'accueil à l'intérieur du dispositif de traitement médical (2) de sorte que le mouvement des buses (14) depuis ladite position rétractée jusqu'à ladite position avancée amène au moins certaines desdites buses (14), en particulier toutes lesdites buses (14), à venir en prise avec des passages de fluide (18) respectifs du dispositif de couplage amovible (4), permettant ainsi au dispositif de traitement médical (2) de fournir le milieu de traitement depuis les buses (14), via les passages de fluide en prise (18) du dispositif de couplage amovible (4), dans tout canal de l'article médical (28) raccordé au dispositif de couplage amovible (4),
dans lequel le dispositif de traitement médical (2) comprend un actionneur (46), dans lequel l'actionneur est configuré pour déplacer les buses (14) entre ladite position rétractée et ladite position avancée,
dans lequel l'actionneur (46) comprend un moteur (48) et une partie d'actionneur mobile (50),
dans lequel le moteur (48) est configuré pour entraîner la partie d'actionneur mobile (50) alternativement dans un mouvement de marche avant et de marche arrière le long d'un premier axe géométrique, **caractérisé en ce que** l'un desdits mouvements de marche avant et de marche arrière de la partie d'actionneur mobile (50) amène les buses (14) à se déplacer transversalement audit premier axe géométrique de la position rétractée à la position avancée, et l'autre desdits mouvements de marche avant et de marche arrière de la partie d'actionneur mobile (50) amène les buses (14) à revenir de la position avancée à la position rétractée.

2. Système (1) selon la revendication 1, dans lequel le dispositif de traitement médical (2) comprend une unité d'accueil (16), l'unité d'accueil (16) comprenant une pluralité de compartiments (30), un pour chaque buse (14), dans lequel
- dans ladite position rétractée, chaque buse (14) est située au moins partiellement à l'intérieur de son compartiment respectif (30), en particulier entièrement à l'intérieur de son compartiment respectif (30), et
- dans ladite position avancée, chaque buse (14) fait saillie hors de son compartiment respectif (30).

3. Système (1) selon la revendication 2, dans lequel ledit emplacement d'accueil pour le dispositif de couplage amovible (4) est aligné avec l'unité d'accueil (16) de sorte que, lorsque le dispositif de couplage amovible (4) est positionné à l'emplacement d'accueil, chaque passage de fluide (18) est aligné coaxialement avec une buse (14) respective.

4. Système (1) selon l'une quelconque des revendications 1 à 3, dans lequel, dans la position avancée des buses (14), les buses (14) sont configurées pour faire saillie dans les passages de fluide (18) du dispositif de couplage amovible (4).

5. Système (1) selon l'une quelconque des revendications 1 à 4, dans lequel chaque buse (14) est pourvue d'au moins un joint d'étanchéité (40, 42) configuré pour suivre le mouvement de la buse (14).

6. Système (1) selon la revendication 2 ou l'une quelconque des revendications 3 à 5 lorsqu'elles dépendent de la revendication 2, dans lequel chaque buse (14) comprend un joint d'étanchéité avant (40) et un joint d'étanchéité arrière (42), ayant chacun une périphérie interne en contact circonférentiel avec la buse (14), dans lequel, lorsque la buse (14) est dans la position avancée et est en prise avec un passage de fluide (18), une périphérie externe du joint d'étanchéité avant (40) s'applique de manière étanche contre une partie de paroi circonférentielle du passage de fluide en prise (18), et une périphérie externe du joint d'étanchéité arrière (42) s'applique de manière étanche contre une partie de paroi circonférentielle du compartiment à partir duquel la buse (14) fait saillie.

7. Système (1) selon l'une quelconque des revendications 1 à 6, dans lequel, dans la position avancée des buses (14), lorsqu'elles sont venues en prise avec les passages de fluide (18), les buses (14) empêchent le dispositif de couplage amovible (4) de se déplacer dans une direction radiale par rapport à l'étendue axiale de l'une quelconque des buses (14).

8. Système (1) selon l'une quelconque des revendications 1 à 7, dans lequel la partie d'actionneur (50) comprend un plan incliné configuré pour interagir avec une partie de maintien de buse, dans lequel le mouvement de la partie d'actionneur (50) dans l'un desdits mouvements de marche avant et de marche arrière amène le plan incliné à pousser la partie de maintien de buse transversalement audit premier axe géométrique, déplaçant ainsi les buses (14) de la position rétractée à la position avancée.

9. Système (1) selon l'une quelconque des revendications 1 à 8, comprenant en outre une unité de commande (60) et un dispositif de détection de position (64) en communication de données avec l'unité de commande (60), dans lequel le dispositif de détection de position (64) est configuré pour indiquer que le dispositif de couplage amovible (4) est positionné audit emplacement d'accueil, dans lequel, lors de la réception d'informations provenant du dispositif de détection de position (64) indiquant que le dispositif de couplage amovible (4) a été positionné audit emplacement d'accueil, l'unité de commande (60) est configurée pour envoyer une demande de mouvement (62) à l'actionneur afin d'amener les buses (14) à se déplacer de la position rétractée à la position avancée de manière à venir en prise avec les passages de fluide (18) du dispositif de couplage amovible (4).

10. Système (1) selon l'une quelconque des revendications 1 à 9, dans lequel chaque passage de fluide (18) présente un côté d'entrée (32) pour recevoir une buse (14) respective et un côté de sortie (34) pour le raccordement aux canaux de l'article médical (28), en particulier dans lequel le côté d'entrée (32) de chaque passage de fluide (18) comprend une cavité (36) pour recevoir une buse (14) respective.

11. Système (1) selon la revendication 10, dans lequel le dispositif de couplage amovible (4) comprend une pluralité d'embouts de raccordement (20) définissant les côtés de sortie (34) des passages de fluide (18), dans lequel les embouts de raccordement (20) sont configurés pour être raccordés auxdits canaux de l'article médical (28) soit directement, soit via des tubes de raccordement (24) inter-raccordant lesdits canaux avec les embouts de raccordement (20).

12. Système (1) selon l'une quelconque des revendications 1 à 11, dans lequel le dispositif de couplage amovible (4) est configuré pour pouvoir être monté de manière amovible sur un support de charge (8) destiné à porter des articles médicaux (28), de sorte que l'insertion du support de charge (8) dans le dispositif de traitement médical (2) amène le dispositif de couplage amovible monté (4) à se positionner audit emplacement d'accueil.

13. Système (1) selon la revendication 12, dans lequel le dispositif de couplage amovible (4) est configuré pour être au moins partiellement mobile lorsqu'il est monté sur le support de charge (8), dans lequel chaque buse (14) est pourvue d'une partie de pointe effilée pour permettre un ajustement fin de la position du dispositif de couplage amovible (4), ledit ajustement fin étant permis en autorisant les parties de pointe des buses (14) à pénétrer dans les passages de fluide (18) pendant l'avancement de la position rétractée à la position avancée même si les buses (14) et les passages de fluide ne sont pas alignés coaxialement lorsque les buses (14) sont déplacées hors de la position rétractée ; en particulier dans lequel le dispositif de couplage amovible (4) est configuré pour être au moins partiellement mobile par rapport au support de charge (8).

14. Système (1) selon l'une quelconque des revendications précédentes, dans lequel les buses (14) avancent chacune le long d'un axe central respectif différent vers l'extérieur en s'éloignant d'une paroi latérale (10) ou d'une unité d'accueil (16) du dispositif de traitement médical (2) lors du déplacement vers la position avancée, en particulier dans lequel lesdits axes centraux respectifs sont mutuellement parallèles.

15. Système (1) selon l'une quelconque des revendications précédentes, dans lequel le dispositif de couplage amovible (4) comprend ou fait partie d'une structure pour maintenir l'article médical (28) lorsque l'article médical (28) est raccordé au dispositif de couplage amovible (4), en particulier dans lequel la structure pour maintenir l'article médical (28) comprend un panier, un bac, une boîte, un plateau ou une plateforme.

16. Système (1) selon l'une quelconque des revendications précédentes, dans lequel le dispositif de couplage amovible (4) est configuré pour être raccordé à un endoscope, et le dispositif de traitement médical (2) comprend un appareil parmi un laveur-désinfecteur d'endoscopes ou un dispositif de séchage d'endoscopes.
